# EUROPEAN PATENT APPLICATION

(11) **EP 4 850 738 A2**
(43) Date of publication of application: **09.12.2026**
(21) Application number: 26211489.5
(22) Date of filing: 05.07.2017
(51) Int. Cl.: A61M 25/06

(54) **INTRODUCER SHEATH FOR VASCULAR ACCESS**

(30) Priority: 06.07.2016 EP 16178169
(62) Document number(s) of the earlier application(s) in accordance with Art. 76 EPC: 17737549.0 / 3 481 480
(71) Applicant: Abiomed Europe GmbH, 52074 Aachen (DE)
(72) Inventor: SIESS, Thorsten, Aachen (DE); ABOULHOSN, Walid, Aachen (DE); KIRCHHOFF, Frank, Aachen (DE)
(74) Representative: Klunker IP Patentanwälte PartG mbB

(57) **Abstract**

An introducer sheath (10) for providing vascular access in a patient's body comprises a tubular body (11) having a proximal end (12) and a distal end (13), the distal end (13) being configured to be inserted into a patient's vessel (50) to allow a medical device (100) to be inserted from the proximal end (12) through the tubular body (11) out of the distal end (13) into the patient's vessel (50). The tubular body (11) includes a recoil section (31) that has a radial compressibility which is higher than a radial compressibility of at least a portion of the tubular body (11) that is distal to the recoil section (31). This enables the patient's vessel (50) and surrounding tissue (51) to recoil at the puncture site (52), which is advantageous for the patient, e.g. with respect to wound healing.

## Description

### BACKGROUND

This invention relates to an introducer sheath for providing vascular access in a patient's body.

Long term vascular access is a common medical procedure used in several medical situations including dialysis for patients requiring frequent dialysis treatments, chemotherapy treatment or ventricular assist device use. Different devices and different methods are used depending on patient needs. Long term vascular access in patients needing ventricular assist devices is common through an open chest procedure and direct cardiovascular access.

Lately there has been a move toward the use of peripheral vessels to access the cardiovascular system in order to avoid traumatic open chest surgery. The move toward the use of peripheral vessels instead of central cardiovascular vessels has been accompanied by the development of a large number of specific devices and tools that are specifically designed for peripheral use. Larger devices have been introduced, therefore requiring larger introducer sheaths. Vascular introducers are the most common devices that have been developed to allow peripheral vascular access. For providing access to a vessel, an introducer sheath usually is directly pierced into a vessel at a puncture site, for example with the help of a dilator.

An introducer sheath for providing vascular access in a patient's body typically comprises a tubular body having a distal end and a proximal end, wherein the distal end is configured to be inserted into a patient's vessel to allow a medical device to be inserted from the proximal end through the tubular body out of the distal end into the patient's vessel. It will be appreciated that the term "proximal" refers to directions towards a user, such as a medical practitioner, while the term "distal" refers to directions away from the user.

The diameter of the introducer sheath is a limiting factor in providing vascular access. On the one hand, the outer diameter is limited depending on the patient and the vessel that is intended to be accessed. Introducer sheaths having diameters that are too large cannot be introduced into the vessel or may cause harm to the patient, which can lead to severe bleeding. Typically, the larger the diameter of an introducer the higher the risk of further patient complications. Therefore, there is a need to minimize the diameter of the introducer sheath especially when the sheath diameter is relatively large. On the other hand, the introducer sheath must provide a minimum inner diameter to allow a medical device such as a catheter or a catheter with an intravascular blood pump to pass therethrough into the patient's vessel. For instance, a medical device with a dimension of 14 French would require an introducer sheath with a dimension of 16 or 17 French. In particular, large diameter introducer sheaths dilate the opening in the vessel and surrounding tissue at the puncture site.

Vascular tissue has a tendency to recoil when punctured, i.e. to return at least partly to its original configuration. This tendency to recoil diminishes if the vascular tissue remains stretched for an extended period of time. Vascular tissue typically recoils to a certain extent depending on the time it was stretched, normally about 2 to 3 French. In addition, the larger the puncture diameter the more recoil is observed. Therefore, in the use of large diameter introducers tissue recoil could be more significant than in the use of small introducers. Especially in long term applications, there is a tendency for the tissue at the puncture site not to recoil, which may lead to bleeding and requires a relatively large wound to be closed.

### SUMMARY OF THE INVENTION

It is therefore an object of the present invention to provide an introducer sheath that reduces harm to the patient's vessel, in particular at the puncture site.

This object is achieved according to the present invention by an introducer sheath and a method defined in the independent claims. Preferred embodiments and further developments of the invention are specified in the claims dependent thereon.

According to the invention, the tubular body of the introducer sheath includes a recoil section that has a radial compressibility which is higher than a radial compressibility of at least a portion of the tubular body that is distal to the recoil section, preferably higher than a radial compressibility of the rest of the tubular body. Radial compressibility refers to the ability of the tubular body to be compressed in a radial direction, wherein a high radial compressibility relates to "soft" characteristics compared to a low radial compressibility that relates to "hard" characteristics. Upon placement of the introducer sheath in the patient's vessel, the recoil section is positioned such that it extends across the puncture site. This configuration has several advantages. Because the tubular body has a recoil section that is placed in the region of the puncture site, the surrounding skin and vessel tissue is allowed to recoil, i.e. return at least partially to its initial configuration. This is allowed by the increased radial compressibility of the recoil section. Thus, the opening at the puncture site can close at least partially, which may improve healing of the puncture site and reduces bleeding potential and further harm to the patient's vessel. Further, the distal end of the tubular body is stiff enough to be inserted into the patient's vessel in the usual way.

Besides reducing harm to the vessel at the puncture site, the recoil section can reduce the risk of bleeding. Due to the increased radial compressibility in the recoil section, the tubular body is allowed to constrict in the region of the recoil section. Therefore, because the portion that is distal to the recoil section does not radially compress, a plug-like effect is achieved that acts on the inner wall of the vessel and seals the opening in the vessel when retracted partially out of the vessel. At the same time, the recoil section may act as an anchor which helps to hold the introducer sheath in the patient's vessel. These effects can be improved if a distal end of the recoil section is formed as a circumferential step or ridge that can lie against tissue at the puncture site from inside the vessel. A proximal end of the recoil section may be smooth in order to facilitate insertion of the introducer sheath, or may also be provided with a step.

Furthermore, the recoil section provides a soft pivot point that allows a medical practitioner, such as a surgeon or cardiologist, to pivot the proximal portion of the tubular body, i.e. substantially the portion of the tubular body that is located outside the patient's body, when the introducer sheath is in place. This provides increased flexibility during use, e.g. in cases where only little space is provided, and the surgeon can move the proximal end of the tubular body in different directions, i.e. can choose the insertion direction by pivoting the proximal end of the tubular body about the recoil section.

The introducer sheath of the present invention allows for recoiling of the puncture site, i.e. permits the opening at the puncture site to constrict. For instance, upon dilating of the puncture site by means of a medical device that is to be inserted through the introducer sheath or by a dilator, tissue at the puncture site can recoil at least partially with the introducer sheath inserted in the patient's vessel after a largest diameter portion of the medical device or the dilator has been retracted from the introducer sheath or advanced through the introducer sheath beyond the puncture site. Again, the tissue surrounding the recoil section may recoil even with the introducer sheath still in place. The tissue may recoil for example by 2 to 3 French. In common introducer sheaths that do not include a recoil section, the tissue at the puncture site cannot recoil as long as the introducer sheath is inserted. The risk of the tissue losing its tendency to recoil after removal of the introducer sheath increases the longer the introducer sheath stays inserted in the patient's vessel.

Preferably, the recoil section is arranged closer to the proximal end than to the distal end of the tubular body. Typically, the introducer sheath will be placed in the patient's vessel such that the puncture site is located in a proximal portion of the tubular body. Since the recoil section is to be placed in the region of the puncture site, it is advantageous if the recoil section is closer to the proximal end of the tubular body than to the distal end. However, in another embodiment, in particular if the part of the tubular body that is inserted into the patient's vessel is shorter than the part of the tubular body that stays outside the patient's body, the recoil section may be arranged closer to the distal end of the tubular body than to the proximal end. In still another embodiment, the recoil section may be equally spaced from both ends, i.e. arranged substantially in the middle between the proximal and distal ends of the tubular body along the length of the tubular body.

According to one embodiment the recoil section has a distal end and a proximal end, wherein the distal end of the recoil section is spaced apart from the distal end of the tubular body and the proximal end of the recoil section is spaced apart from the proximal end of the tubular body. In other words, the recoil section is spaced apart from both ends of the tubular body. Consequently, both the part of the tubular body that is distal to the recoil section and the part of the tubular body that is proximal to the recoil section have a radial compressibility that is less than the radial compressibility of the recoil section. In other words, the recoil section forms a soft section with respect to radial compressibility that is surrounded by harder or stiffer portions of the tubular body. Preferably, the recoil section extends in a longitudinal direction of the tubular body only in an area of the tubular body that is configured to be placed in a region of a puncture site of the patient's vessel.

According to another embodiment the recoil section extends from the proximal end of the tubular body towards the distal end of the tubular body. In contrast to the embodiment described above, the recoil section may alternatively be disposed directly at the proximal end of the tubular body. However, in this embodiment as well, the recoil section is arranged along the length of the tubular body such that it can be positioned in the region of the puncture site. Increasing the length of the recoil section enables a medical practitioner to choose the insertion depth more flexibly. Since the proximal end of the tubular body is not inserted into the patient's vessel, the part of the tubular body that extends up to the proximal end either may have a radial compressibility like the recoil section or may have a lower radial compressibility like the distal portion of the tubular body.

In order to aid in positioning the introducer sheath, at least one of the proximal and distal ends of the recoil section, preferably both ends, may be marked with a radiopaque material. This enables a medical practitioner to observe the position of the introducer sheath under x-ray. The radiopaque material may be provided e.g. in the form of metal rings or additives in the material of the tubular body. Another feature that may be helpful for correct placement of the introducer sheath may be provided in the form of pressure sensing means. For instance, a first lumen may be provided that extends from the proximal end of the tubular body to the distal end of the recoil section and exits the tubular body there. Thus, once the distal end of the recoil section enters the blood vessel, blood enters the lumen and can be detected from the outside. Preferably, a second lumen extends from the proximal end of the tubular body to the proximal end of the recoil section and exits the tubular body there. Thus, a pressure difference between the distal end and the proximal end of the recoil section can be determined. If the pressure is the same or substantially the same, the introducer sheath is inserted too far into the blood vessel because both lumens are in fluid communication with the blood vessel. Once the second lumen, which exits at the proximal end of the recoil section, does not indicate a blood pressure, while the first lumen, which exits at the distal end of the recoil section, does indicate a blood pressure, the introducer sheath is correctly placed with the recoil section disposed in the area of the puncture site.

Advantageously, the recoil section extends in a circumferential direction of the tubular body uniformly around the tubular body, i.e. extends completely and continuously about the circumference of the tubular body. However, the recoil section may be formed non-uniformly in a direction along the circumference of the tubular body as long as the increased radial compressibility is ensured. For instance, the recoil section may comprise first and second portions that may be arranged in an alternating manner about the circumference of the tubular body, wherein the first portions provide the increased radial compressibility and the second portions have substantially the same characteristics as the rest of the tubular body. This may result in non-uniform behavior and in particular in non-circular cross-sections of the tubular body, when the recoil section is radially compressed. Furthermore, the second portions provide axial stiffness of the tubular body, which is advantageous in particular during insertion of the introducer sheath into the patient's vessel. For example, there may be two first portions and two second portions, although any number may be envisioned. The first end second portions may have the same size or different sizes, in particular with respect to a circumferential direction of the tubular body.

The radial compressibility of the recoil section which is higher than the radial compressibility of at least the portion of the tubular body that is distal to the recoil section can be achieved by varying of the geometry, in particular the wall thickness of the tubular body, or by varying of the material of tubular body, or both.

According to one embodiment, the recoil section has a wall thickness that is smaller than a wall thickness of a portion of the tubular body distally adjacent to the recoil section. Preferably, the recoil section has a wall thickness that is smaller than a wall thickness of the rest of the tubular body. The recoil section of the tubular body may have a wall thickness of 0.3 mm or less, preferably of less than 0.2 mm, more preferably of less than 0.15 mm, even more preferably of less than 0.1 mm. In this embodiment in particular, the recoil section preferably is made of or consists of the same material as the rest of the tubular body or at least the portion of the tubular body that is distal to the recoil section.

According to another embodiment, the recoil section has a wall thickness that is substantially equal to a wall thickness of the rest of the tubular body. The recoil section is then preferably formed of a material having a rigidity that is less than a rigidity of a material of the rest of the tubular body or at least the portion of the tubular body that is distal to the recoil section.

The introducer sheath may be configured for introducing a medical device into a patient's vessel. The medical device may for instance comprise a catheter, and may preferably comprise an intravascular blood pump arranged at a distal end of the catheter. The tubular body may have a length of about 10 cm to about 30 cm, preferably about 20 cm. The diameter of the tubular body may be in the range from about 12 French to about 18 French, preferably 14 French. The tubular body is preferably made of polyethylene. Other suitable materials such as PTFE may be chosen alternatively.

### BRIEF DESCRIPTION OF THE DRAWINGS

The foregoing summary, as well as the following detailed description of preferred embodiments, will be better understood when read in conjunction with the appended drawings. For the purpose of illustrating the present disclosure, reference is made to the drawings. The scope of the disclosure is not limited, however, to the specific embodiments disclosed in the drawings. In the drawings:
Fig. 1A shows an introducer set including an introducer sheath and a dilator in an assembled configuration.
Fig. 1B shows the introducer sheath and the dilator of Fig. 1A separated from each other.
Fig. 2A shows an introducer set according to another embodiment including an introducer sheath and a dilator in an assembled configuration.
Fig. 2B shows the introducer sheath and the dilator of Fig. 2A separated from each other.
Fig. 3 shows an embodiment of an introducer sheath.
Fig. 4 shows another embodiment of an introducer sheath.
Fig. 5 shows still another embodiment of an introducer sheath.
Fig. 6 shows yet another embodiment of an introducer sheath.
Fig. 7 shows a detail of another embodiment of an introducer sheath.
Fig. 8 shows a detail of another embodiment of an introducer sheath.
Fig. 9 shows a detail of still another embodiment of an introducer sheath.
Fig. 10 shows a perspective view of a tubular sheath according to another embodiment.
Fig. 11 shows an introducer sheath inserted into a patient's blood vessel.
Fig. 12 shows an application of the introducer sheath.
Fig. 13 shows another application of the introducer sheath.

### DETAILED DESCRIPTION

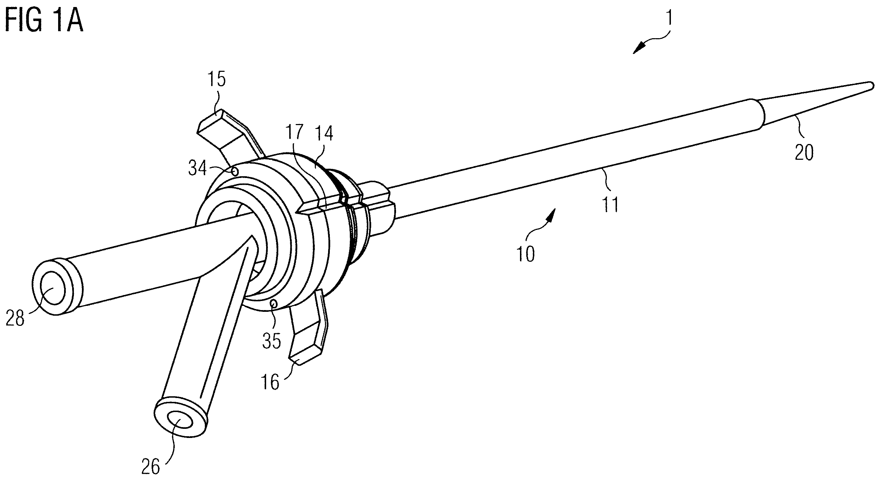

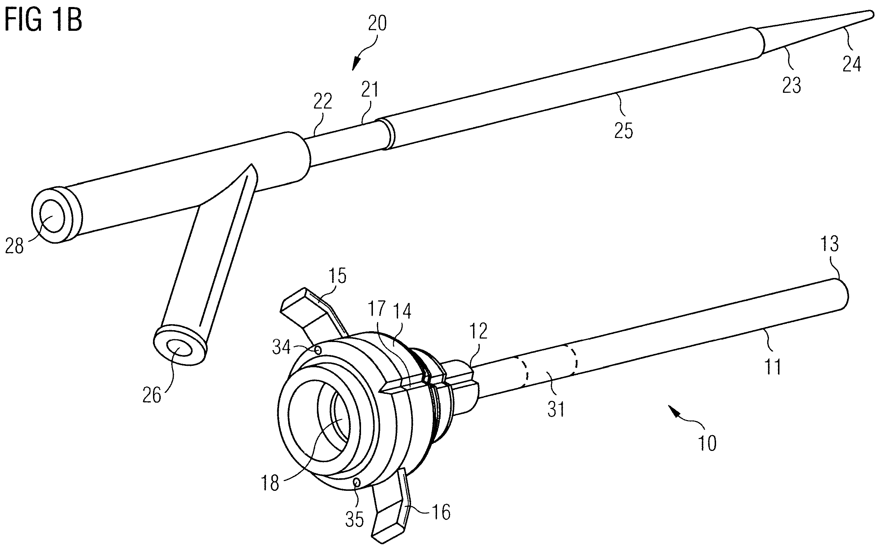

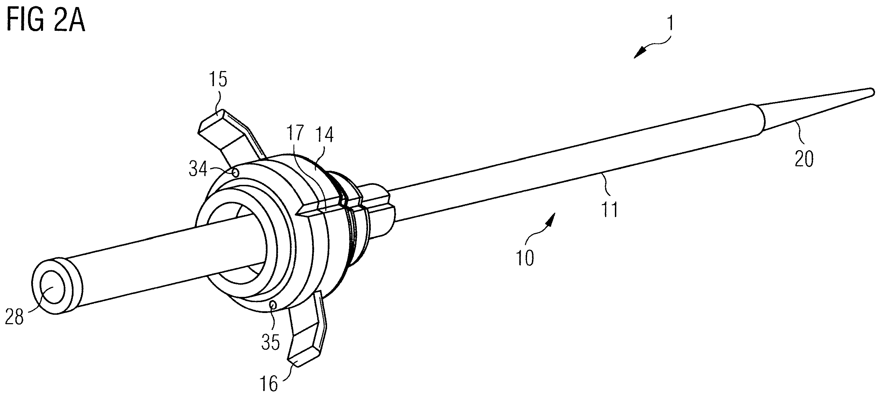

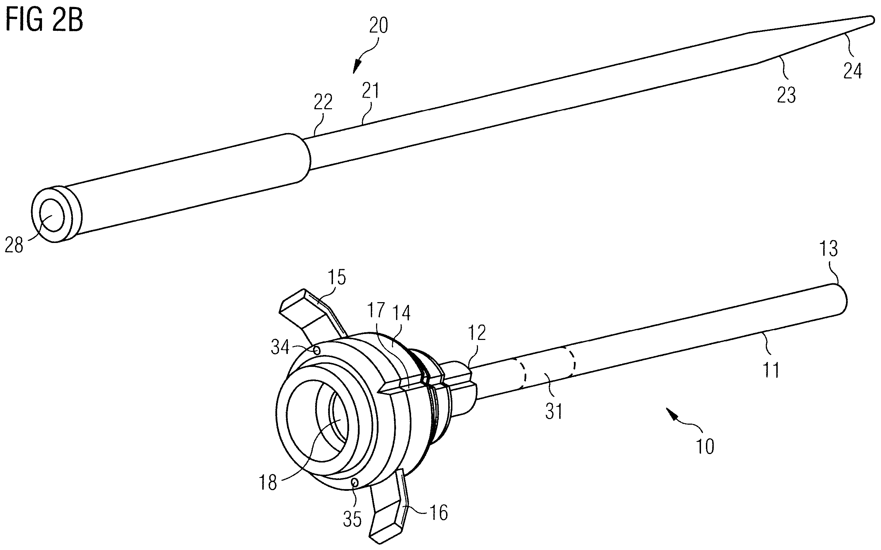

Referring to Figs. 1A to 2B, different views of an introducer set 1 are shown. The introducer set 1 includes an introducer sheath 10 and a dilator 20, which are shown assembled in Figs. 1A and 2A, respectively, and separately in Figs. 1B and 2B respectively. The introducer sheath 10 includes a tubular body 11 having a proximal end 12 and a distal end 13. A hemostasis valve 14 having a flexible membrane 18 is provided at the proximal end 12. However, it will be appreciated that the hemostasis valve may be constructed differently or in some applications a hemostasis valve can be omitted. In this exemplary embodiment of the hemostasis valve 14, two handles 15 and 16 are provided for manipulating the introducer sheath 10 and possibly for separating the introducer sheath 10 if necessary or desired, in particular splitting the hemostasis valve 14. A longitudinal notch 17 is provided in the hemostasis valve 14 to form a predetermined breaking line. Lumens 34, 35 are provided that extend through the valve 14 and the tubular body 11 as described in more detail below.

The shown dilators 20 are exemplary dilators. It will be appreciated that any other suitable dilator may be used in combination with the introducer sheath of the present invention, in particular a dilator without a balloon. The dilator 20 has a body 21 with a proximal portion 22 and a distal portion 23. The distal portion 23 includes a tapered tip 24 to facilitate insertion into the patient's vessel. In the embodiment of Figs. 1A and 1B ports 26 and 28 are provided at the proximal portion 22. The port 28 is connected to an internal lumen that is configured to receive a guide wire therethrough (not shown). In other words, the dilator 20 can be placed over a guide wire that has been inserted into the patient's vessel using the Seldinger technique for example. A balloon 25 is disposed on the body 21 of the dilator 20 and extends over a major portion thereof, excluding the tapered tip 24 and the distal-most portion of the body 21. The balloon 25 can be inflated and deflated via the port 26 which is connected to the balloon 25 via a lumen 27 in the proximal portion 22 of the body 21. The balloon 25 is made of a non-compliant material such as Nylon.

The balloon 25 of the dilator 20 is utilized to support the introducer sheath 10 during insertion into the patient's vessel. The dilator 20 is inserted into the introducer sheath 10 with the balloon 25 deflated. In other words, the balloon is preloaded before operation. A fluid, such as air, is filled into the balloon 25 via the port 26 to inflate the balloon 25 such that the balloon 25 contacts an inner surface of the introducer sheath 10. Surface friction between the dilator 20 and the introducer sheath 10 is thereby increased, which provides stability to the introducer sheath 10 during insertion into the patient's vessel and prevents the introducer sheath 10 from buckling. When the dilator 20 is to be retracted after the assembly has been inserted into the patient's vessel, the balloon 25 is deflated to decrease surface friction between the dilator 20 and the introducer sheath 10. The dilator 20 can then be retracted from the introducer sheath 10 substantially without any interference. The dilator 20 and the introducer sheath 10 can move freely. As mentioned above and shown in Figs. 2A and 2B, a dilator 20 without a balloon can be provided alternatively, which, apart from the balloon, is constructed equally or similarly to the dilator 20 shown in Figs. 1A and 1B. It will be appreciated that the introducer sheath 10 may be sufficiently stiff such that it will not be always necessary to provide support by means of a balloon or the like.

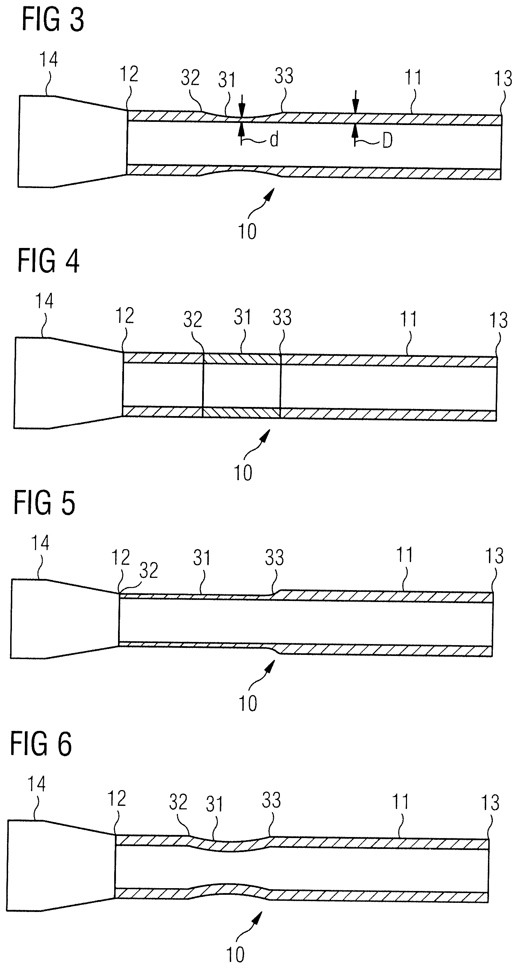

Figs. 3 to 9 show different embodiments of an introducer sheath 10 having a tubular body 11 with a recoil section 31, i.e. a section with increased radial compressibility compared to the rest of the tubular body 11 or at least compared to a portion of the tubular body 11 that is distal with respect to the recoil section 31. Figs. 3 to 9 each show an introducer sheath 10 with a tubular body 11 with a proximal end 12 and a distal end 13. A hemostasis valve 14 is schematically shown at the proximal end 12 of the tubular body 11. As explained above, the hemostasis valve 14 can be omitted or other means for preventing bleeding may be provided.

In the embodiment shown in Fig. 3, the recoil section 31 is formed of a portion of the tubular body 11 having a wall thickness d that is smaller than a wall thickness D of the rest of the tubular body 11. This provides increased radial compressibility in the recoil section 31. The wall thickness d may be 0.3 mm or less. Preferably, the variation of the wall thickness of the tubular body 11 is achieved by varying of the outer diameter of the tubular body 11. In particular, the outer diameter of the tubular body 11 may be reduced in the recoil section 31, while the inner diameter remains constant along the length of the tubular body 11. It will be appreciated that, alternatively, the inner diameter may be increased in the recoil section 31 while the outer diameter remains constant. The variation of the wall thickness of the tubular body 11 may also be a combination thereof. In this embodiment, the tubular body 11 is integrally formed of a single material, e.g. made of a single layer of one material.

The recoil section 31 has a proximal end 32 that is spaced from the proximal end 12 of the tubular body 11, and a distal end 33 that is spaced from the distal end 13 of the tubular body 11. That means that the tubular body 11 of the introducer sheath 10 generally provides a relatively high stiffness and can be easily handled. Only the recoil section 31, which is the section of the tubular body 11 that will be positioned in an area of a puncture site of a blood vessel, has a reduced stiffness (i.e. an increased radial compressibility) to enable tissue at the puncture site to recoil, i.e. to return at least partially to its initial structure.

In the embodiment shown in Fig. 4, the wall thickness of the tubular body 11 is substantially constant along the length of the tubular body 11. However, in order to provide a recoil section 31 with increased radial compressibility, the recoil section 31 is made of a material that has a stiffness that is less than a stiffness of a material of the rest of the tubular body 11. It will be appreciated that the embodiments of Figs. 3 and 4 may be combined, i.e. the recoil section 31 may be formed by a different wall thickness, in particular a smaller wall thickness, and a different material, in particular a softer material, compared to the rest of the tubular body 11.

In the embodiment of Fig. 5, the recoil section 31 does not only extend in an area that is spaced from the proximal and distal ends 12, 13 of the tubular body 11. The recoil section 31 extends from the proximal end 12 of the tubular body 11 towards the distal end 13 of the tubular body 11. In other words, the proximal end 32 of the recoil section 31 coincides with the proximal end 12 of the tubular body 11, whereas the distal end 33 of the recoil section 31 is spaced apart from the distal end 13 of the tubular body 11. The stiffness of the part of the introducer sheath 10 outside the patient's body is less crucial than the stiffness of the part that is placed within the blood vessel. Increasing the length of the recoil section 31 enables a medical practitioner to choose the insertion depth more flexibly. In this embodiment, the recoil section 31 is formed of a portion of the tubular body 11 having a reduced wall thickness, similar to the embodiment of Fig. 3. It will be appreciated that alternatively or in addition, the tubular body 11 may have a constant wall thickness with the recoil section 31 made from a different and softer material that is more compressible than the tubular body 11 like in the embodiment of Fig. 4.

In the embodiment shown in Fig. 6, the recoil section 31 forms a portion of the tubular body 11 which has a reduced inner diameter. In other words, in contrast to the aforementioned embodiments, the inner diameter of the tubular body 11 is not constant along its length. Further, it will be appreciated that in any of the shown embodiments inner diameter of the tubular sheath 11 need not be constant along its length. The recoil section 31 may be formed in accordance with any of the embodiments of Fig. 3 or Fig. 4 for example, that is to say the increased radial compressibility either may be achieved by varying the geometry or the material of the tubular body 11 or both.

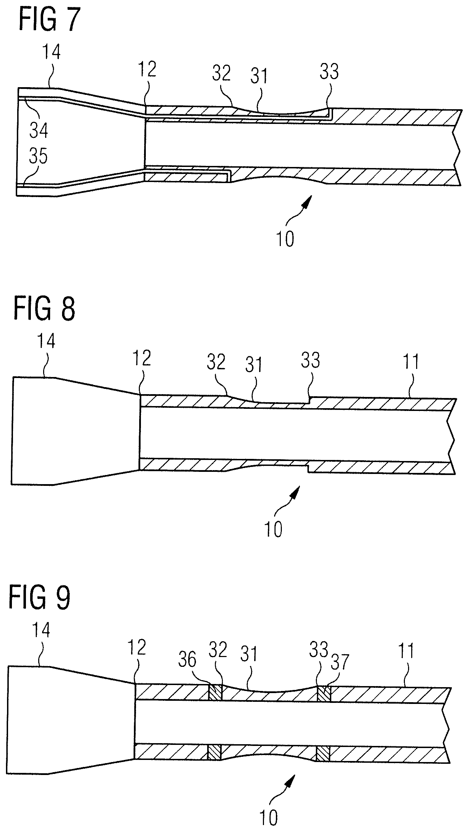

In the introducer sheath 10 of the embodiment shown in Fig. 7, a first lumen 34 and a second lumen 35 are provided, which has been briefly mentioned in connection with Fig. 1. The first lumen 34 extends from the proximal end of the introducer sheath 10, such as the hemostatic valve 14, into the tubular body 11 past the proximal end 32 of the recoil section 31 and exits the tubular body 11 substantially at the distal end 33 of the recoil section 31. The lumen 34 is used to sense a pressure, e.g. by means of an appropriate pressure sensor. Blood enters the lumen 34 once the introducer sheath 10 is inserted far enough such that the distal end 33 of the recoil section 31 is placed in fluid communication with the patient's vessel. The second lumen 35 is similar to the first lumen 34 but exits the tubular body 11 substantially at the proximal end 32 of the recoil section 31. If both the proximal end 32 and the distal end 33 of the recoil section 31 are disposed inside the patient's vessel, substantially no pressure difference will be detected. A pressure difference between the proximal end 32 and the distal end 33 will be detected when the proximal end 32 is outside the patient's body and the distal end 33 is inside the patient's vessel. This indicates that the introducer sheath 10 is positioned correctly.

Fig. 8 shows an embodiment that is similar in particular to the embodiment of Fig. 3. However, the recoil section 31 at its distal end 33 does not transition into the distal portion of the tubular body 11 smoothly but forms a steep transition, e.g. in the form of a step or ridge. This aids in preventing the introducer sheath 10 from backing out of the patient's vessel because the step forms a stop for a movement of the introducer sheath 10 in a distal direction. The transition of the recoil section 31 at the proximal end 32 is smooth to facilitate insertion of the introducer sheath 10 into the patient's vessel. However, it will be appreciated that both the proximal end 32 and the distal end 33 may be formed as a step.

Another embodiment is shown in Fig. 9 which is substantially similar to the aforementioned embodiments and may be combined with any of the described embodiments. In order to help a medical practitioner in positioning the introducer sheath 10 correctly in the patient's vessel, i.e. with the recoil section 31 in the region of the puncture site, the proximal end 32 and the distal end 33 of the recoil section 31 may be marked by means of a radiopaque material, such as metal rings 36, 37 or radiopaque additives in the material of the tubular body 11. It will be appreciated that only one of the ends 32, 33 can be marked with a radiopaque material or the entire recoil section 31 may be marked, e.g. by providing a radiopaque material in the wall of the tubular body 11 in the region of the recoil section 31.

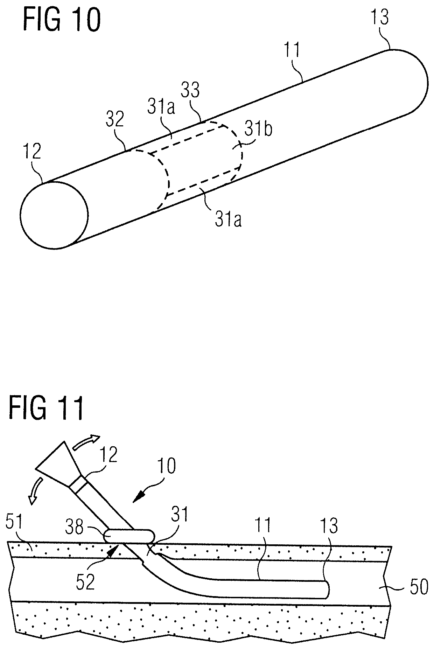

Fig. 10 shows a perspective view of a tubular body 11 of an introducer sheath according to another embodiment. In contrast to the other embodiments, the recoil section 31 is not formed uniformly about the circumference of the tubular body 11 but comprises different portions 31a, 31b. The portions 31a, 31b may be arranged in an alternating manner, e.g. two of the portions 31a and two of the portions 31b. The portions 31a, 31b may be equally sized or may have a different size. For instance, the portions 31a may have the same or substantially the same radial compressibility as the rest of the tubular body 11, thus providing stiffness and stability for the tubular body 11, in particular in an axial direction. The portions 31b may have an increased radial compressibility, e.g. by providing a thinner wall or softer material in this area compared to the rest of the tubular body 11. This results in a non-circular compression of the recoil section 31.

Referring to Fig. 11, an introducer sheath 10 is schematically shown that is inserted in a patient's blood vessel 50. The distal end 13 of the tubular body 11 is positioned within the vessel 50, whereas the proximal end 12 stays outside the patient's body. The introducer sheath 10 is inserted into the blood vessel 50 through a puncture site 52 and through other tissue 51, such as skin tissue. The recoil section 31 enables recoiling of the tissue 51 and of the opening in the blood vessel 50 at the puncture site 52, i.e. the tubular body 11 is constricted in the region of the recoil section 31 by forces applied by the surrounding tissue. Recoiling of the tissue 51 and the resulting narrowing of the tubular body 11 at the recoil section 31 also reduces the risk of bleeding because the narrowed shape of the tubular body 11 provides a plug effect. Further, as indicated by arrows in Fig. 11, the recoil section 31 provides a soft pivot point about which the part of the introducer sheath 10 that is proximal to the recoil section 31 can be pivoted to some extent, which allows a medical practitioner to more flexibly choose the insertion direction from which a medical device, such as a catheter with an intravascular blood pump, is inserted into the blood vessel 50 through the introducer sheath 10.

The recoil section 31 will maintain a circular or substantially circular shape and abut the puncture site 52 due to the higher blood pressure inside the recoil section 31 relative to the atmosphere. Alternatively, in particular in view of the embodiment of Fig. 10, the recoil section 31 may be formed of softer and harder portions resulting in a non-circular shape, such as ellipsoidal, which may promote pivoting in a certain direction. As shown in Fig. 11, a stop 38, such as a rubber ring or the like, may be provided to prevent the introducer sheath 10 from slipping into the vessel 50. The stop 38 is advantageously disposed at the proximal end 32 of the recoil section 31 in order to aid in positioning the introducer sheath 10.

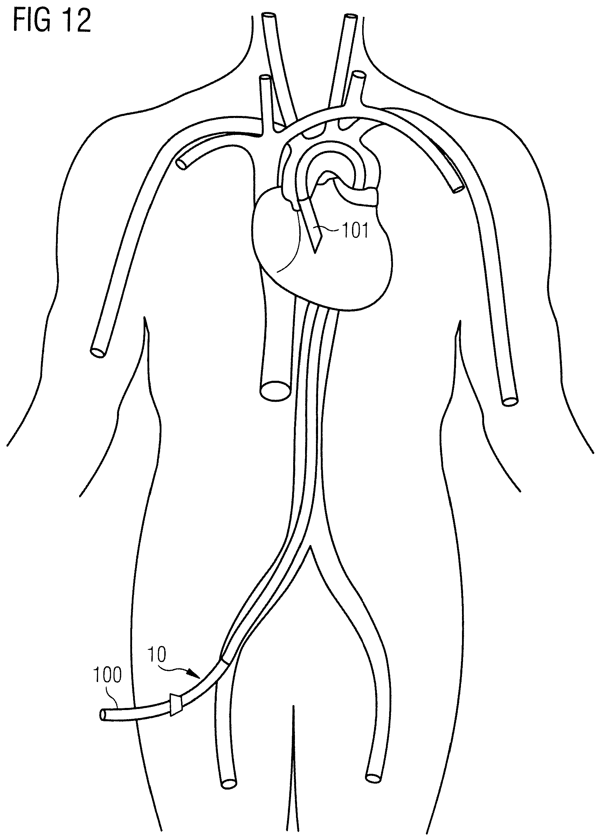

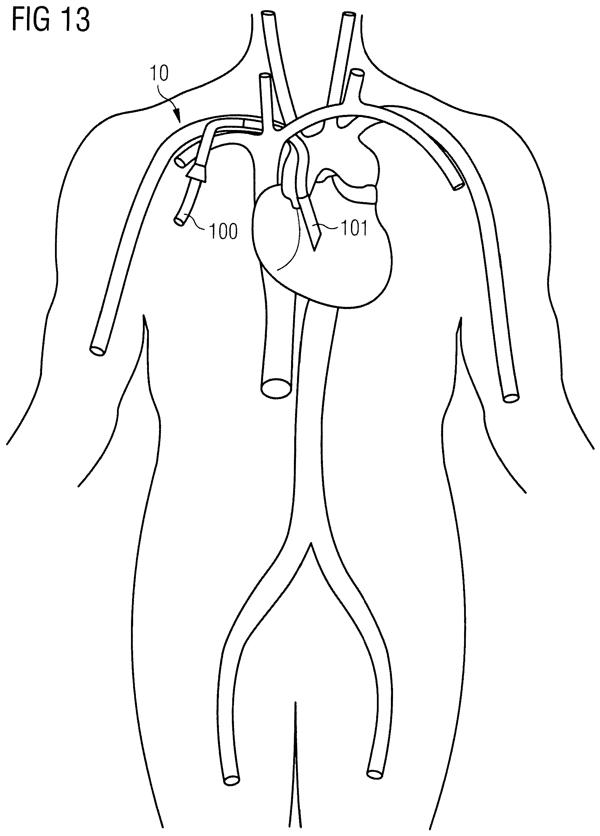

Referring now to Figs. 12 and 13, applications of an introducer sheath 10 are shown. The introducer sheath 10 may be in accordance with any one of the above disclosed embodiments. It is used to insert an intravascular blood pump 101 by means of a catheter 100 through a patient's vessel into the patient's heart to provide a ventricular assistant device. The vascular access may be placed in a peripheral vessel in the patient's groin (Fig. 12) or in the patient's thorax (Fig. 13).
Preferred embodiments of the invention are described in the following numbered paras 1 to 15:
1. An introducer sheath (10) for providing vascular access in a patient's body, comprising a tubular body (11) having a proximal end (12) and a distal end (13), the distal end (13) being configured to be inserted into a patient's vessel to allow a medical device (100) to be inserted from the proximal end (12) through the tubular body (11) out of the distal end (13) into the patient's vessel, wherein the tubular body (11) includes a recoil section (31) that has a radial compressibility which is higher than a radial compressibility of at least a portion of the tubular body (11) that is distal to the recoil section (31).
2. The introducer sheath of para 1, wherein the recoil section (31) is arranged closer to the proximal end (12) than to the distal end (13) of the tubular body (11).
3. The introducer sheath of para 1 or 2, wherein the recoil section (31) has a proximal end (32) and a distal end (33), wherein the distal end (33) of the recoil section (31) is spaced apart from the distal end (13) of the tubular body (11) and the proximal end (32) of the recoil section (31) is spaced apart from the proximal end (12) of the tubular body (11), wherein the recoil section (31) preferably extends in a longitudinal direction of the tubular body (11) only in an area of the tubular body (11) that is configured to be placed in a region of a puncture site of the patient's vessel.
4. The introducer sheath of para 1 or 2, wherein the recoil section (31) extends from the proximal end (12) of the tubular body (11) towards the distal end (13) of the tubular body (11).
5. The introducer sheath of any one of paras 1 to 4, wherein the recoil section (31) has a proximal end (32) and a distal end (33), wherein at least one of the proximal end (32) and the distal end (33) is marked with a radiopaque material.
6. The introducer sheath of any one of paras 1 to 5, wherein the recoil section (31) extends in a circumferential direction of the tubular body (11) uniformly around the tubular body (11).
7. The introducer sheath of any one of paras 1 to 6, wherein the recoil section (31) has a wall thickness (d) that is smaller than a wall thickness (D) of a portion of the tubular body (11) distally adjacent to the recoil section (31).
8. The introducer sheath of any one of paras 1 to 7, wherein the recoil section (31) has a wall thickness (d) that is smaller than a wall thickness (D) of the rest of the tubular body (11).
9. The introducer sheath of any one of paras 1 to 8, wherein the recoil section (31) has a wall thickness (d) of 0.3 mm or less, preferably of less than 0.2 mm, more preferably of less than 0.15 mm, even more preferably of less than 0.1 mm.
10. The introducer sheath of any one of paras 1 to 9, wherein the recoil section (31) consists of the same material as the rest of the tubular body (11).
11. The introducer sheath of any one of paras 1 to 6, wherein the recoil section (31) has a wall thickness that is substantially equal to a wall thickness of the rest of the tubular body (11).
12. The introducer sheath of any one of paras 1 to 11, wherein the recoil section (31) is made of a material having a rigidity that is less than a rigidity of a material of the rest of the tubular body (11).
13. An introducer set, comprising the introducer sheath of any one of paras 1 to 12 and a medical device (100, 101), wherein the introducer sheath (10) is configured for introducing the medical device (100, 101) into a patient's vessel (50), wherein the medical device comprises a catheter (100), and preferably an intravascular blood pump (101) arranged at a distal end of the catheter (101).
14. A method of providing vascular access in a patient's body, comprising the step of placing an introducer sheath (10) through a puncture site (52) into a patient's vessel (50), the introducer sheath (10) comprising a tubular body (11) having a proximal end (12) and a distal end (13), the distal end (13) being configured to be inserted into a patient's vessel (50) to allow a medical device (100) to be inserted from the proximal end (12) through the tubular body (11) out of the distal end (13) into the patient's vessel (50), wherein the tubular body (11) includes a recoil section (31) that has a radial compressibility which is higher than the radial compressibility of at least a portion of the tubular body (11) that is distal to the recoil section (31),
   wherein the method comprises the step of placing the introducer sheath (10) in the patient's vessel (50) such that the recoil section (31) extends across the puncture site (52).
15. The method of para 14, further comprising the step of dilating the puncture site (52) by means of the medical device (101) or by a dilator (20), allowing tissue at the puncture site (52) to recoil at least partially with the introducer sheath (10) inserted in the patient's vessel (50) after a largest diameter portion of the medical device (101) or the dilator (20) has been retracted from the introducer sheath (10) or advanced through the introducer sheath (10) past the puncture site (50).

## Claims

1. An introducer sheath (10) for providing vascular access in a patient's body, comprising a tubular body (11) having a proximal end (12) and a distal end (13), the distal end (13) being configured to be inserted into a patient's vessel to allow a medical device (100) to be inserted from the proximal end (12) through the tubular body (11) out of the distal end (13) into the patient's vessel, wherein the tubular body (11) includes a recoil section (31) that has a radial compressibility which is higher than a radial compressibility of at least a portion of the tubular body (11) that is distal to the recoil section (31).

2. The introducer sheath of claim 1, wherein the recoil section (31) is arranged closer to the proximal end (12) than to the distal end (13) of the tubular body (11).

3. The introducer sheath of claim 1 or 2, wherein the recoil section (31) has a proximal end (32) and a distal end (33), wherein the distal end (33) of the recoil section (31) is spaced apart from the distal end (13) of the tubular body (11) and the proximal end (32) of the recoil section (31) is spaced apart from the proximal end (12) of the tubular body (11), wherein the recoil section (31) preferably extends in a longitudinal direction of the tubular body (11) only in an area of the tubular body (11) that is configured to be placed in a region of a puncture site of the patient's vessel.

4. The introducer sheath of claim 1 or 2, wherein the recoil section (31) extends from the proximal end (12) of the tubular body (11) towards the distal end (13) of the tubular body (11).

5. The introducer sheath of any one of claims 1 to 4, wherein the recoil section (31) has a proximal end (32) and a distal end (33), wherein at least one of the proximal end (32) and the distal end (33) is marked with a radiopaque material.

6. The introducer sheath of any one of claims 1 to 5, wherein the recoil section (31) extends in a circumferential direction of the tubular body (11) uniformly around the tubular body (11).

7. The introducer sheath of any one of claims 1 to 6, wherein the recoil section (31) has a wall thickness (d) that is smaller than a wall thickness (D) of a portion of the tubular body (11) distally adjacent to the recoil section (31).

8. The introducer sheath of any one of claims 1 to 7, wherein the recoil section (31) has a wall thickness (d) that is smaller than a wall thickness (D) of the rest of the tubular body (11).

9. The introducer sheath of any one of claims 1 to 8, wherein the recoil section (31) has a wall thickness (d) of 0.3 mm or less, preferably of less than 0.2 mm, more preferably of less than 0.15 mm, even more preferably of less than 0.1 mm.

10. The introducer sheath of any one of claims 1 to 9, wherein the recoil section (31) consists of the same material as the rest of the tubular body (11).

11. The introducer sheath of any one of claims 1 to 6, wherein the recoil section (31) has a wall thickness that is substantially equal to a wall thickness of the rest of the tubular body (11).

12. The introducer sheath of any one of claims 1 to 11, wherein the recoil section (31) is made of a material having a rigidity that is less than a rigidity of a material of the rest of the tubular body (11).

13. An introducer set, comprising the introducer sheath of any one of claims 1 to 12 and a medical device (100, 101), wherein the introducer sheath (10) is configured for introducing the medical device (100, 101) into a patient's vessel (50), wherein the medical device comprises a catheter (100), and preferably an intravascular blood pump (101) arranged at a distal end of the catheter (101).

14. A method of providing vascular access in a patient's body, comprising the step of placing an introducer sheath (10) through a puncture site (52) into a patient's vessel (50), the introducer sheath (10) comprising a tubular body (11) having a proximal end (12) and a distal end (13), the distal end (13) being configured to be inserted into a patient's vessel (50) to allow a medical device (100) to be inserted from the proximal end (12) through the tubular body (11) out of the distal end (13) into the patient's vessel (50), wherein the tubular body (11) includes a recoil section (31) that has a radial compressibility which is higher than the radial compressibility of at least a portion of the tubular body (11) that is distal to the recoil section (31),
wherein the method comprises the step of placing the introducer sheath (10) in the patient's vessel (50) such that the recoil section (31) extends across the puncture site (52).

15. The method of claim 14, further comprising the step of dilating the puncture site (52) by means of the medical device (101) or by a dilator (20), allowing tissue at the puncture site (52) to recoil at least partially with the introducer sheath (10) inserted in the patient's vessel (50) after a largest diameter portion of the medical device (101) or the dilator (20) has been retracted from the introducer sheath (10) or advanced through the introducer sheath (10) past the puncture site (50).
